# EUROPEAN PATENT SPECIFICATION

(11) **EP 2 075 066 B1**
(45) Date of publication and mention of the grant of the patent: **17.08.2016**
(21) Application number: 08170895.0
(22) Date of filing: 05.12.2008
(51) Int. Cl.: B01J 27/08, B01J 27/128, B01J 35/00, B01J 37/02, B01J 37/04, C07C 17/25, C07C 21/18

(54) **Method for making catalyst compositions of alkali metal halide-doped bivalent metal fluorides and a process for making fluorinated olefins**
Verfahren zum Herstellen von Katalysatorzusammensetzungen von mit Alkalimetallhalogenid dotierten zweiwertigen Metallfluoriden und Verfahren zur Herstellung fluorierter Olefine
Procédé de fabrication de compositions de catalyseurs de fluorures métalliques divalents dopés à l'halogénure métallique alcalin et procédé de fabrication d'oléfines fluorées

(30) Priority: 10.12.2007 US 12566 P; 21.11.2008 US 275656
(43) Date of publication of application: 01.07.2009
(60) Divisional application: 13153852.2
(73) Proprietor: Honeywell International Inc., Morristown, NJ 07962 (US)
(72) Inventor: Wang, Haiyou, P.O. Box 22445 Morristown, NJ 07962-2245 (US); Tung, Hsueh Sung, Getzville, NY 14068 (US)
(74) Representative: Stepney, Gregory John

(56) References cited:
- WO-A1-2009/009421
- WO-A2-2009/018561
- RU-C2- 2 277 068
- US-A- 4 113 655
- US-A- 4 194 990

## Description

### BACKGROUND OF THE INVENTION

### 1. Field of the Invention

The present invention relates to a method for making catalyst compositions of alkali metal halide-doped bivalent metal fluorides. The catalyst compositions can be used in a process for making fluorinated olefins.

### 2. Description of the Related Art

2,3,3,3-tetraflouropropene (1234yf), a hydrofluoroolefin that exhibits low global warming potential, can be used in a variety of applications, for example, as a refrigerant, a blowing agent, a solvent, a cleaning agent, and a monomer for macromolecular compounds.

One process for making 1234yf entails the dehydrochlorination of 1,1,1,2-tetrafluoror-2-chloropropane (244bb). U.S. Provisional Application 60/958,468, filed July 6, 2007, discloses a process for making 1234yf by dehydrochlorinating 244bb in the presence of catalysts of bivalent metal fluorides doped with alkali metal halides.

US-4,194,990 discloses catalysts and processes for the production of chlorofluorinated hydrocarbons by reacting a hydrocarbon with HCl, HF and an oxygen-containing gas under oxychlorofluorination conditions in the presence of a catalytic composition comprising magnesium and copper ions each in combination with fluoride ions and an alkali metal ion, or mixtures thereof, in combination with chloride ions. RU-2,227,068 C2 discloses the production of lithium-containing fluorides for electrolytic production of aluminium by sorption of lithium from natural salting liquor followed by desorption of it with water, thus obtaining a solution containing LiCl with CaCl₂ and MgCl₂ at ratios dictated by composition of salting liquor. The solution is then concentrated, treated with water and Li, Mg and Ca carbonates settle, after which they are subjected to treatment with hydrofluoric acid for obtaining mixture of LiF, MgF₂ and CaF₂.

There is a need for commercially viable methods for preparing catalysts of bivalent metal fluorides doped with alkali metal halides.

### SUMMARY OF THE INVENTION

According to the present invention, there is provided a method for making a catalyst composition. The catalyst composition is represented by the formula MX/M'F₂. MX is an alkali metal halide wherein M is Cs⁺; X is Cl⁻; the bivalent metal fluoride is represented by the formula M'F_{2;} and M' is selected from the group consisting of Mg²⁺ and Ni²⁺.

According to the present invention, there is provided a method for making a catalyst composition. The method has the following steps: (a) an amount of hydroxide, oxide, or carbonate of the alkali metal defined above is added to an aqueous solution of a hydrogen halide and reacted to form an aqueous solution of alkali metal halide; (b) an amount of a hydroxide, oxide, or carbonate of a bivalent metal defined above is added to an aqueous solution of hydrogen fluoride and reacted to form a precipitate of a bivalent metal fluoride therein; (c) admixing the alkali metal halide solution and the precipitate of the bivalent metal fluoride to form an aqueous slurry; and (d) removing water from the aqueous slurry to form a solid mass.

### DETAILED DESCRIPTION OF THE INVENTION

Catalyst compositions that are useful products of the methods of the present invention are combinations/admixtures of an alkali metal halide(s) and a bivalent metal fluoride(s) that can be represented by the following:

MX/M'F₂

wherein M is Cs⁺; X is Cl⁻; and M' is selected from the group consisting of Mg²⁺ and Ni²⁺. M' is most preferably Mg²⁺.

The catalyst compositions can alternately be represented by the following:

n% MX/M'F₂

wherein n% is the weight percentage of alkali metal halide in the composition based upon the total weight of the composition. The alkali metal halide is preferably from 0.05 wt% to 50 wt%, more preferably 5 wt% to 15 wt%, and most preferably 7.5 wt% to 12.5 wt% of the catalyst composition based on the total weight of the catalyst composition.

The bi-valent metal fluoride is added to the solution of the alkali metal halide to form a slurry. After formation of the slurry, substantially all of the solvent is removed to form a solid mass of a mixture of the alkali metal halide and bi-valent metal fluoride. Although the solvent can be removed in one step, a preferred method is to drive off a portion of the solvent from the slurry to form a paste and then follow by drying the paste to form the solid mass. Any conventional technique can be used to drive off the solvent. Examples of such techniques include vigorous stirring at room or elevated temperatures, evaporation, settling and decanting, centrifugation, and filtration. It is preferred to evaporate off a desired amount of solvent to form the paste. The paste is then dried by any suitable method to form a free-flowing, substantially solvent-free powder. Preferred methods for drying include oven drying, most preferably at temperatures from 110 °C to 120 °C, and spray drying. Being solvent free means that less than 1 wt.%, preferably 0.5 wt% or less, more preferably 0.1 wt% or less, and most preferably no solvent will remain with the powder after solvent removal/drying. Upon removal of solvent, the powder will take the form of a solid mass (or powder) of a mixture of particles of the alkali metal halide and the bi-valent metal fluoride.

In the method of the present invention, a slurry of the alkali metal halide and the bivalent metal fluoride is prepared by a reactive technique. In a first step, a hydroxide, oxide, or carbonate of an alkali metal is added to an aqueous solution of a hydrogen halide and reacted to form an aqueous solution of an alkali metal halide. In a second step, a hydroxide, oxide, or carbonate of a bivalent metal is added to an aqueous solution of hydrogen fluoride and reacted to form a precipitate of a bivalent metal fluoride therein. In a third step, the alkali metal halide solution and the bivalent metal fluoride precipitate are then admixed to form an aqueous slurry. In a fourth step, water is then removed from the aqueous slurry in the manner described herein to form a solid mass.

Optionally, the solid mass of the mixture of the alkali metal halide and the bivalent metal fluoride powder is then calcined. Calcination is preferably carried out at a temperature of 100 °C to 750 °C, more preferably at a temperature of 200 °C to 600 °C, and most preferably at a temperature of 300 °C to 600 °C. Calcination may further optionally be carried out in the presence of an inert gas, such as nitrogen or argon.

After calcination, the powder is optionally further grinded such that it becomes more finely-divided. The powder is further optionally pelletized in order to form pellets. The pellets then provide catalyst surfaces to use in actual process application.

The catalyst compositions of the present invention may afford performance advantages over compositions that are obtained by simple dry mixing of components. A more complete degree of intermixing may be achieved. The complete degree of mixing may manifest itself in higher selectivity to the target product, such as 1234yf (and less to the formation of a dehydrofluorinating product, such as 1233xf).

The catalyst compositions are useful in converting hydrochlorofluorocarbons to fluorinated olefins. Useful hydrochlorofluorocarbons have at least one hydrogen and at least one chlorine on adjacent carbons.

Table 1 sets forth examples of fluorinated olefins and precursor hydrochlorofluorocarbons from which they can be obtained (precursor hydrochlorofluorocarbon in left column and corresponding product fluorinated olefin in the right column).

**Table 1**

| Hydrochlorofluorocarbon | Fluorinated Olefin(s) |
|---|---|
| chlorotetrafluoropropane | tetrafluoropropene |
| chloropentafluoropropane | pentafluoropropene |
| chlorohexafluoropropane | hexafluoropropene |
| 1,1,1,2-tetrafluoro-2-chloropropane CF₃CFClCH₃ (244bb) | 2,3,3,3-tetrafluoropropene CF₃CF=CH₂ (1234yf) |
| 1,1,1,2-tetrafluoro-3-chloropropane CF₃CHFCH₂Cl (244eb) | 2,3,3,3-tetrafluoropropene CF₃CF=CH₂ (1234yf) |
| 1,1,1,3-tetrafluoro-3-chloropropane CF₃CH₂CHFCl (244fa) | 1,3,3,3-tetrafluoropropene CF₃CH=CHF (trans/cis-1234ze) |
| 1,1,1,3-tetrafluoro-2-chloropropane CF₃CHClCH₂F (244db) | 1,3,3,3-tetrafluoropropene CF₃CH=CHF (trans/cis-1234ze) |
| 1,1,1,2,3-pentafluoro-2-chloropropane CF₃CFClCH₂F (235bb) | 1,2,3,3,3-pentafluoropropene CF₃CF=CHF (Z/E-1225ye) |
| 1,1,1,2,3-pentafluoro-3-chloropropane CF₃CHFCHFCl (235ea) | 1,2,3,3,3-pentafluoropropene CF₃CF=CHF (Z/E-1225ye) |
| 1,1,1,3,3-pentafluoro-3-chloropropane CF₃CH₂CF₂Cl (235fa) | 1,1,3,3,3-pentafluoropropene CF₃CH=CF₂ (1225zc) |
| 1,1,1,3,3-pentafluoro-2-chloropropane CF₃CHClCHF₂ (235da) | 1,1,3,3,3-pentafluoropropene CF₃CH=CF₂ (1225zc) |
| 1,1,1,2,3,3-hexafluoro-2-chloropropane CF₃CFClCHF₂ (226ba) | 1,1,2,3,3,3-hexafluoropropene CF₃CF=CF₂ (1216) |
| 1,1,1,2,3,3-hexafluoro-3-chloropropane CF₃CHFCF₂Cl (226ea) | 1,1,2,3,3,3-hexafluoropropene CF₃CF=CF₂ (1216) |

The following examples show the benefit of catalysts that can be produced according to the claimed invention. (Note that only the exemplified CsCl-based catalysts can be made according to the currently claimed invention).

### EXAMPLES

In the following examples, 244bb is dehydrochlorinated to 1234yf in the presence of the catalysts of combinations of alkali metal halides and bivalent metal fluorides.

### Example 1: 244bb Dehydrohalogenation over CsCl/MgF₂ Catalysts having Various CsCl Loadings

A series of CsCl/MgF₂ catalysts with various loadings of CsCl were tested to determine the effect of CsCl loading on reactivity. 20 cc of catalyst pellets was typically used. A mixture of 97.2%/2.0% 244bb/1233xf was passed through catalyst bed at a rate of 6 g/h (grams/hour) at a temperature ranging from 470 °C to 520 °C. The temperatures at the bottom and top of the catalyst bed were measured. As shown in Table 2 below, activity remained at about the same level regardless of loading, while the selectivity to 1233xf (a non-desired dehydrofluorination product) decreased as CsCl loading increased to 5.0 wt%. No 1233xf was formed over the 10 wt% CsCl/MgF₂ catalyst.

**Table 2**

| (Effect of CsCl loading on the performance of CsCl/MgF₂ catalysts during 244bb dehydrohalogenation*) | | | | | | |
|---|---|---|---|---|---|---|
| CsCl loading (wt%) | Temp. Bottom-Top (°) | time (h) | Conversion, (%) 244bb | Selectivity(% ) 1234yf | Selectivity(% ) 1233xf | Selectivity(% ) others |
| 0.0 | 475-506 | 1 | 48.2 | 76.9 | 17.7 | 5.4 |
| | 475-509 | 2 | 52.9 | 79.8 | 14.6 | 5.6 |
| | 475-509 | 3 | 53.3 | 80.7 | 12.9 | 6.4 |
| | 475-507 | 4 | 52.4 | 81.4 | 11.9 | 6.7 |
| | 475-509 | 5 | 54.2 | 83.0 | 10.9 | 6.1 |
| | 475-510 | 6 | 54.1 | 83.6 | 10.2 | 6.2 |
| | 475-508 | 7 | 54.7 | 84.7 | 9.6 | 5.7 |
| | 475-509 | 8 | 53.7 | 85.4 | 9.2 | 5.4 |
| | 475-510 | 9 | 54.9 | 86.0 | 8.6 | 5.5 |
| | 475-509 | 10 | 53.5 | 86.7 | 8.2 | 5.1 |
| 2.5 | 500-514 | 1 | 48.4 | 88.7 | 5.2 | 6.1 |
| | 500-514 | 2 | 48.1 | 88.5 | 5.2 | 6.3 |
| | 500-514 | 3 | 49.5 | 89.1 | 5.0 | 5.9 |
| | 500-507 | 4 | 46.9 | 89.3 | 4.8 | 5.9 |
| | 500-509 | 5 | 48.5 | 89.9 | 4.6 | 5.5 |
| | 500-513 | 6 | 48.5 | 89.6 | 4.7 | 5.7 |
| | 500-514 | 7 | 49.6 | 89.9 | 4.6 | 5.5 |
| 5.0 | 490-510 | 1 | 49.0 | 94.8 | 0.5 | 4.7 |
| | 490-511 | 2 | 51.0 | 94.5 | 0.4 | 5.1 |
| | 490-510 | 3 | 49.2 | 95.3 | 0.5 | 4.2 |
| | 490-505 | 4 | 48.7 | 95.0 | 0.4 | 4.6 |
| | 490-507 | 6 | 49.8 | 95.4 | 0.4 | 4.2 |
| | 490-503 | 8 | 49.2 | 95.7 | 0.4 | 3.9 |
| 10.0 | 475-511 | 1 | 49.6 | 96.9 | | 3.1 |
| | 475-510 | 2 | 51.2 | 97.0 | | 3.0 |
| | 475-511 | 3 | 51.8 | 96.9 | | 3.1 |
| | 475-508 | 4 | 50.4 | 96.9 | | 3.1 |
| | 475-510 | 5 | 51.4 | 97.0 | | 3.0 |
| • Reaction conditions: 20 ml of catalyst, 6 grams organic/hour, 97.2% 244bb/2.0% 1233xf, 101325 Pa (1 atm) pressure | | | | | | |

### Example 2: 244bb Dehydrohalogenation over 10 wt% Alkali Metal Chloride/MgF₂ Catalysts

10 wt% KCl/MgF₂ and 10 wt% CsCl/MgF₂ catalysts were tested. 20 cc of catalyst pellets was used. A mixture of 99.1%/0.4% 244bb/1233xf was passed through the catalyst bed at a rate of 6 g/h at a temperature ranging from 380 °C to 480 °C. The temperature at the bottom and top of the catalyst bed were measured. As shown in Table 2, both catalysts exhibited about the same activity (20%), while the 10 wt% CsCl/MgF₂ catalyst provided a higher selectivity to 1234yf without generation of 1233xf over the catalyst.

**Table 3**

| (Reactivity of 10 wt% KCl/MgF₂ and 10 wt% CsCl/MgF₂ Catalysts during 244bb Dehydrohalogenation*) | | | | | | |
|---|---|---|---|---|---|---|
| Catalyst | Temp. Bottom-Top (°) | time (hour) | Conversion (%) 244bb | Selectivity(%) 1234yf | Selectivity(%) 1233xf | Selectivity(%) others |
| 10 wt% KCl/MgF2 | 405-477 | 1 | 21.9 | 89.1 | 0.4 | 10.5 |
| | 405-480 | 2 | 17.8 | 95.2 | 0.6 | 4.2 |
| | 405-480 | 4 | 20.0 | 95.8 | 0.6 | 3.6 |
| | 405-480 | 6 | 21.2 | 96.0 | 0.6 | 3.7 |
| | 405-480 | 8 | 20.1 | 96.1 | 0.6 | 3.3 |
| | 405-480 | 10 | 21.5 | 96.2 | 0.6 | 3.1 |
| | 405-480 | 12 | 20.9 | 96.2 | 0.6 | 3.2 |
| | 405-479 | 14 | 20.5 | 96.3 | 0.6 | 3.1 |
| | 405-479 | 16 | 20.2 | 96.2 | 0.6 | 3.2 |
| | 405-479 | 18 | 20.1 | 96.3 | 0.6 | 3.1 |
| | 405-480 | 20 | 20.5 | 96.4 | 0.6 | 3.0 |
| | 405-478 | 22 | 20.4 | 96.4 | 0.6 | 3.0 |
| | 405-478 | 24 | 19.9 | 96.3 | 0.6 | 3.1 |
| 10 wt% CsCl/MgF₂ | 380-481 | 1 | 10.3 | 91.1 | 0.0 | 8.9 |
| | 380-481 | 2 | 14.0 | 95.9 | 0.0 | 4.1 |
| | 380-482 | 4 | 16.8 | 96.7 | 0.0 | 3.3 |
| | 380-484 | 6 | 19.6 | 97.4 | 0.0 | 2.6 |
| | 380-482 | 8 | 20.0 | 97.5 | 0.0 | 2.5 |
| | 380-481 | 10 | 20.5 | 97.5 | 0.0 | 2.5 |
| | 380-481 | 12 | 20.6 | 97.8 | 0.0 | 2.2 |
| | 380-479 | 14 | 19.9 | 97.7 | 0.0 | 2.3 |
| | 380-478 | 16 | 20.0 | 97.8 | 0.0 | 2.2 |
| | 380-481 | 14 | 19.9 | 97.7 | 0.0 | 2.3 |
| | 380-483 | 16 | 20.0 | 97.8 | 0.0 | 2.2 |
| | 380-481 | 18 | 21.0 | 97.8 | 0.0 | 2.2 |
| | 380-481 | 20 | 21.8 | 98.0 | 0.0 | 2.0 |
| | | 22 | 20.7 | 97.7 | 0.0 | 2.3 |
| | | 24 | 19.7 | 97.6 | 0.0 | 2.4 |
| • Reaction conditions: 20 ml of catalyst, 6 grams organic/hour, 99.1%/0.4% 244bb/1233xf, 101325 Pa (1 atm) pressure | | | | | | |

### Example 3: 244bb Dehydrohalogenation over 10 wt% CsCl/Bi-Valent Metal Fluoride Catalysts

10 wt% CsCl/NiF₂ and 10 wt% CsCl/MgF₂ catalysts were tested. 20 cc of catalyst pellets was used. A mixture of 99.1%/0.4% 244bb/1233xf was passed through a catalyst bed at a rate of 6 g/h at a temperature ranging from 380 °C to 480 °C. The temperature at the bottom and top of the catalyst bed were measured. As shown in Table 4, both catalysts exhibited about the same selectivity to 1234yf (97% to 98%), while the 10 wt% CsCl/MgF₂ catalyst provided higher activity.

**Table 4**

| (Reactivity of MgF₂ and Alkaline Metal Chloride-Doped MgF₂ Catalysts during 244bb Dehydrohalogenation*) | | | | | | |
|---|---|---|---|---|---|---|
| Catalyst | Temp. Bottom-Top (°) | time (hour) | Conversion (%) 244bb | Selectivity (%) 1234yf | Selectivity (%)1233xf | Selectivity (%) others |
| 10 wt% CsCl/NiF₂ | 410-482 | 1 | 5.6 | 86.3 | 0.0 | 13.7 |
| | 410-482 | 2 | 8.3 | 90.4 | 0.0 | 9.6 |
| | 410-483 | 4 | 9.9 | 93.6 | 0.0 | 6.4 |
| | 410-480 | 6 | 10.1 | 95.2 | 0.0 | 4.8 |
| | 410-481 | 8 | 10.9 | 95.9 | 0.0 | 4.1 |
| | 410-480 | 10 | 12.0 | 96.2 | 0.0 | 3.8 |
| | 410-481 | 12 | 13.2 | 96.8 | 0.0 | 3.2 |
| | 410-482 | 14 | 14.2 | 97.1 | 0.0 | 2.9 |
| | 410-481 | 16 | 14.4 | 97.3 | 0.0 | 2.7 |
| | 410-481 | 18 | 14.7 | 97.3 | 0.0 | 2.7 |
| | 410-480 | 20 | 14.8 | 97.4 | 0.0 | 2.6 |
| | 410-481 | 22 | 15.1 | 97.8 | 0.0 | 2.2 |
| | 410-480 | 24 | 15.4 | 97.6 | 0.0 | 2.4 |
| 10 wt% CsCl/MgF₂ | 380-481 | 1 | 10.3 | 91.1 | 0.0 | 8.9 |
| | 380-481 | 2 | 14.0 | 95.9 | 0.0 | 4.1 |
| | 380-482 | 4 | 16.8 | 96.7 | 0.0 | 3.3 |
| | 380-484 | 6 | 19.6 | 97.4 | 0.0 | 2.6 |
| | 380-482 | 8 | 20.0 | 97.5 | 0.0 | 2.5 |
| | 380-481 | 10 | 20.5 | 97.5 | 0.0 | 2.5 |
| | 380-481 | 12 | 20.6 | 97.8 | 0.0 | 2.2 |
| | 380-479 | 14 | 19.9 | 97.7 | 0.0 | 2.3 |
| | 380-478 | 16 | 20.0 | 97.8 | 0.0 | 2.2 |
| | 380-481 | 18 | 21.0 | 97.8 | 0.0 | 2.2 |
| | 380-483 | 18 | 21.0 | 97.8 | 0.0 | 2.2 |
| | 380-481 | 20 | 21.8 | 98.0 | 0.0 | 2.0 |
| | 380-481 | 22 | 20.7 | 97.7 | 0.0 | 2.3 |
| | | 24 | 19.7 | 97.6 | 0.0 | 2.4 |
| * Reaction conditions: 20 ml of catalyst, 6 grams organic/hour, 99.1 % 244bb/0.4% 1233xf, 101325 Pa (1 atm) pressure | | | | | | |

### Example 4: 244bb dehydrohalogenation over alkaline metal chloride-doped MgF₂ catalysts

In Example 4, a series of alkaline metal chlorides were investigated as an additive to MgF₂ with a purpose of improving the selectivity to 1234yf. For comparison purpose, the results obtained over MgF₂ catalyst were also reported. 20 cc of catalyst pellets was used in a typical run. A mixture of 97.2% 244bb / 2.0% 1233xf was passed through catalyst bed at a rate of 6 g/h (grams/hour) at a temperature ranged from 470°C to 520°C. The temperatures at the bottom of catalyst bed and at the top of catalyst bed were measured.

As shown in Table 5, the MgF₂ provided a 244bb conversion of 53-55%, a 1234yf selectivity of 80-87%, and a 1233xf selectivity of 8-15%; the 10% LiCl/MgF₂ provided a 244bb conversion below 45%, a 1234yf selectivity of about 90%, and a 1233xf selectivity of about 5%; the 10% KCl/MgF₂ provided a 244bb conversion below 50%, a 1234yf selectivity of about 96%, and a 1233xf selectivity of about 1%; and the 10% CsCl/MgF₂ provided a 244bb conversion of 50-52%, a 1234yf selectivity of about 97%, and no formation of 1233xf. CsCl exhibited the best results. The 10% CsCl/MgF₂ catalyst provided activity comparable to MgF₂ and the highest 1234yf selectivity while generating no 1233xf.

**Table 5**

| (Reactivity of alkaline metal chloride-doped MgF₂ catalysts during 244bb dehydrohalogenation*) | | | | | | |
|---|---|---|---|---|---|---|
| Catalyst | Temp. Bottom-Top (°) | t (h) | Conversion 244bb (%) | Selectivity 1234yf (%) | Selectivity 1233xf (%) | Selectivity, Unknowns (%) |
| 10 wt% LiCl/MgF₂ | 475-490 | 1 | 29.4 | 89.1 | 5.3 | 5.6 |
| | 475-506 | 2 | 38.8 | 89.6 | 5.3 | 5.0 |
| | 475-505 | 3 | 40.4 | 89.9 | 5.2 | 4.9 |
| | 475-507 | 4 | 42.9 | 90.5 | 4.8 | 4.7 |
| 10 wt% KCl/MgF2 | 475-514 | 1 | 38.3 | 95.1 | 0.9 | 4.0 |
| | 475-515 | 3 | 47.2 | 95.6 | 0.8 | 3.6 |
| | 475-515 | 5 | 47.5 | 95.8 | 0.7 | 3.5 |
| | 475-509 | 6 | 43.7 | 95.8 | 0.6 | 3.5 |
| | 475-514 | 7 | 47.1 | 95.8 | 0.7 | 3.5 |
| 10 wt% CsCl/MgF₂ | 475-511 | 1 | 49.6 | 96.9 | | 3.1 |
| | 475-510 | 2 | 51.2 | 97.0 | | 3.0 |
| | 475-511 | 3 | 51.8 | 96.9 | | 3.1 |
| | 475-508 | 4 | 50.4 | 96.9 | | 3.1 |
| | 475-510 | 5 | 51.4 | 97.0 | | 3.0 |
| * Reaction conditions: 20 ml of catalyst, 6 grams-organic/hour, 97.2% 244bb / 2.0% 1233xf, pressure = 101325 Pa (1 atm) | | | | | | |

Additional teachings to catalyst compositions having mixtures/combinations of alkali metal halides and bivalent metal fluorides and use of same in dehydrochlorinating hydrofluorocarbons to fluorinated olefins is shown in U.S. Provisional Patent Application No. 60/958,468, filed July 6, 2007.

## Claims

1. A method for making a catalyst composition, comprising:
(a) adding an amount of hydroxide, oxide, or carbonate of an alkali metal to an aqueous solution of a hydrogen halide and reacting to form an aqueous solution of an alkali metal halide;
(b) adding an amount of hydroxide, oxide or carbonate of a bivalent metal to an aqueous solution of hydrogen fluoride and reacting to form a precipitate of a bivalent metal fluoride;
(c) admixing the alkali metal halide solution and the bivalent metal fluoride precipitate to form an aqueous slurry; and
(d) removing water from the aqueous slurry to form a solid mass,
wherein the alkali metal halide is represented by the formula MX; M is Cs⁺; X is Cl⁻; the bivalent metal fluoride is represented by the formula M'F₂; and M' is selected from the group consisting of Mg²⁺ and Ni²⁺.

2. The method of claim 1, wherein the alkali metal halide is from about 0.05 wt% to about 50 wt% of the catalyst composition based on the total weight of the catalyst composition.

3. The method of claim 1, wherein M'F₂ comprises MgF₂.

## Patentansprüche

1. Verfahren zur Herstellung einer Katalysatorzusammensetzung, bei dem man:
(a) eine Menge von Hydroxid, Oxid oder Carbonat eines Alkalimetalls zu einer wässrigen Lösung eines Halogenwasserstoffs gibt und zur Reaktion bringt, wobei man eine wässrige Lösung eines Alkalimetallhalogenids erhält;
(b) eine Menge von Hydroxid, Oxid oder Carbonat eines zweiwertigen Metalls zu einer wässrigen Lösung von Fluorwasserstoff gibt und zur Reaktion bringt, wobei man einen Niederschlag eines Fluorids eines zweiwertigen Metalls erhält;
(c) die Alkalimetallhalogenid-Lösung und den Niederschlag des Fluorids eines zweiwertigen Metalls mischt, wobei man eine wässrige Aufschlämmung erhält; und
(d) Wasser aus der wässrigen Aufschlämmung entfernt, wobei man eine feste Masse erhält,
wobei das Alkalimetallhalogenid durch die Formel MX wiedergegeben wird; M für Cs⁺ steht; X für Cl⁻ steht; das Fluorid eines zweiwertigen Metalls durch die Formel M'F₂ wiedergegeben wird und M' aus der Gruppe bestehend aus Mg²⁺ und Ni²⁺ ausgewählt ist.

2. Verfahren nach Anspruch 1, bei dem das Alkali-metallhalogenid etwa 0,05 Gew.-% bis etwa 50 Gew.-% der Katalysatorzusammensetzung, bezogen auf das Gesamtgewicht der Katalysatorzusammensetzung, ausmacht.

3. Verfahren nach Anspruch 1, bei dem M'F₂ MgF₂ umfasst.

## Revendications

1. Procédé de préparation d'une composition de catalyseur, comprenant :
(a) ajouter une quantité d'hydroxyde, d'oxyde ou de carbonate d'un métal alcalin à une solution aqueuse d'halogénure d'hydrogène et faire réagir afin de former une solution aqueuse d'un halogénure de métal alcalin ;
(b) ajouter une quantité d'hydroxyde, d'oxyde ou de carbonate d'un métal divalent à une solution aqueuse de fluorure d'hydrogène et faire réagir de façon à former un précipité d'un fluorure de métal divalent ;
(c) combiner la solution d'halogénure de métal alcalin et le précipité de fluorure de métal divalent afin de former une suspension aqueuse ; et
(d) éliminer l'eau de la suspension aqueuse afin de former une masse solide,
dans lequel l'halogénure de métal alcalin est représenté par la formule MX ; M est Cs⁺ ; X est Cl⁻ ; le fluorure de métal divalent est représenté par la formule M'F₂ ; et M' est sélectionné dans le groupe constitué de Mg²⁺ et Ni²⁺.

2. Procédé selon la revendication 1, dans lequel l'halogénure de métal alcalin constitue d'environ 0,05 % en poids à environ 50 % en poids de la composition de catalyseur sur la base du poids total de la composition de catalyseur.

3. Procédé selon la revendication 1, dans lequel M'F₂ comprend MgF₂.
